# EUROPEAN PATENT APPLICATION

(11) **EP 4 585 699 A1**
(43) Date of publication of application: **16.07.2025**
(21) Application number: 22957784.6
(22) Date of filing: 09.09.2022
(51) Int. Cl.: C12Q 1/6848, C12Q 1/68, C07D 407/14, C07D 403/02, C07D 249/08, C07D 233/54

(54) **USE OF HETEROAROMATIC RING COMPOUNDS IN NUCLEIC ACID TESTS**

(71) Applicant: BGI Shenzhen, Shenzhen, Guangdong 518083 (CN)
(72) Inventor: ZHUO, Shitian, Shenzhen, Guangdong 518083 (CN); TENG, Bo, Shenzhen, Guangdong 518083 (CN); SHEN, Liang, Shenzhen, Guangdong 518083 (CN); YAN, Shengyi, Shenzhen, Guangdong 518083 (CN); YANG, Qinping, Shenzhen, Guangdong 518083 (CN); YE, Zhiyan, Shenzhen, Guangdong 518083 (CN); XU, Xun, Shenzhen, Guangdong 518083 (CN); ZHANG, Wenwei, Shenzhen, Guangdong 518083 (CN); CHEN, Ao, Shenzhen, Guangdong 518083 (CN)
(74) Representative: Lavoix
(86) International application number: PCT/CN2022/118107
(87) International publication number: WO 2024/050815

(57) **Abstract**

Provided is the use of heteroaromatic ring compounds in nucleic acid tests. Particularly, provided are the use of heteroaromatic ring compounds as a nucleic acid protective agent in nucleic acid tests, a method for inhibiting degradation of nucleic acids during nucleic acid tests, a method for detecting a target nucleic acid molecule, and a method for determining a target single-stranded polynucleotide sequence. The present invention further relates to a reagent and a kit containing the heteroaromatic ring compounds.

## Description

### Technical field

The invention is in the field of nucleic acid detection, and relates to the use of heteroaromatic ring compounds in nucleic acid detection. Particularly, the invention relates to the use of heteroaromatic ring compounds as a nucleic acid protective agent in nucleic acid detection, a method for inhibiting degradation of nucleic acids, a method for detecting a target nucleic acid molecule, and a sequencing method. The invention further relates to a reagent and a kit comprising the heteroaromatic ring compounds.

### Background art

With the rapid development of gene sequencing technology, the second generation high-throughput sequencing technology and single molecule sequencing technology overcome the defects of high cost, long time consumption and the like of Sanger sequencing, and greatly promote the application of gene sequencing technology in various research fields.

However, in the course of sequencing and other processing of nucleic acid samples, nucleic acids are continuously exposed to various harmful conditions from the environment, resulting in continuous damage and degradation of nucleic acid samples, affecting their further processing and test. For example, in the course of sequencing, the damage and degradation of nucleic acids hinder the extension of second strand of DNA, and also lead to an increase in sequencing error rate. With the extension of sequencing read length, the damages superimpose and become more obvious.

A large number of studies have shown that the environmental damage factors faced by nucleic acid in the course of sequencing and other processing mainly come from the laser irradiation of fluorescence detection. The continuous strong irradiation of the excitation light source and the strong EM radiation from the fluorophore with shorter wavelength and higher energy will produce reactive oxygen species in aqueous solution, such as singlet oxygen, superoxide anion and iron-promoted hydroxyl radical. It is these highly reactive radicals that directly or indirectly lead to dimerization of pyrimidines and oxidative modification of nucleotides, even cleavage of phosphate bonds, and ultimately hinder further processing and test of nucleic acid samples, which is specifically manifested in loss of signal intensity of fluorescence detection, high error rate and the like.

In order to ensure high quality sequencing of long nucleotide sequences, it is particularly important to provide an efficient and low-cost nucleic acid protection strategy.

The current main nucleic acid protection strategy is to inhibit light-induced degradation of nucleic acid sequences by the addition of antioxidants. The commonly used antioxidants include vitamin C derivatives and polyphenolic compounds. These antioxidants interact with oxygen free radicals and the like around the environment through their highly reactive hydroxyl groups in the structure, to achieve the effect of quenching free radicals and thus protecting nucleic acid sequences. However, due to their high reactivity, these antioxidants may gradually interact with oxygen in the air during storage and use to degrade and deteriorate, thereby affecting the final use effect. In addition, the poor water solubility of most polyphenolic compounds limits their use in aqueous systems.

### Contents of the invention

An object of the invention is to provide a method for reducing damage to a nucleic acid sample. The technical solution of the invention comprises introducing an additive in the course of treatment, processing, test, reaction and the like of a nucleic acid sample. The additive can fully contact with the nucleic acid sample, and achieve the effect of protecting the nucleic acid sample by quenching free radicals and other harmful factors in the sample environment, absorbing strong EM radiation with short wavelength and high energy and the like.

To achieve the effect, the additive used preferably have one or more of the following characteristics: it has good water solubility to ensure full contact with a nucleic acid sample in a water medium; it is inert in chemical reaction, and does not react with active groups in nucleic acid, so that the normal functional form of the sample can be maintained; it can strongly interact with free radicals and quench harmful factors; it has good absorption effect on strong EM radiation with short wavelength and high energy; its aqueous solution is colorless and transparent, and does not affect the detection of fluorescent luminescence signals and the like.

The inventors found that a class of heteroaromatic ring compounds can achieve the above effect, thereby providing the following invention:

### Use of heteroaromatic ring compounds as a nucleic acid protective agent in nucleic acid detection

In one aspect, the invention provides use of a heteroaromatic ring compound as a nucleic acid protective agent in a nucleic acid detection, wherein the heteroaromatic ring compound is selected from a compound shown in formula (I) or an isomer, salt or N-oxide thereof, or a solvate (such as hydrate) of a compound shown in formula (I) or an isomer, salt or N-oxide thereof; 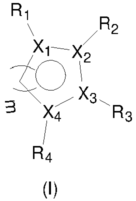
wherein, X₁, X₂, X₃ each are independently selected from C and N; X₄ is selected from N and S; and, at least one of X₁, X₂, X₃ and X₄ is N;
m is 1 or 2;
R₁ is absent or is selected from H, C₁₋₆ alkyl, amino, hydroxy, C₂₋₆ alkenyl, C₂₋₆ alkynyl, and C₁₋₆ alkoxy;
R₂ is absent or is selected from -(CH₂)ₙ-COOH or -(CH₂)ₙ-CONH₂; n is 0, 1, 2, 3 or 4; optionally, -(CH₂)ₙ- is substituted by amino; optionally, the amino is substituted by - (C=O)-(CH₂)_{q}-NH₂; q is 0, 1, 2, 3 or 4;
R₃ is absent or is H;
or, R₂ and R₃ form a 5- to 6-membered heteroaryl group comprising 1-2 nitrogen atoms, the heteroaryl is optionally substituted by 1 or 2 substituents, and the substituents each are independently selected from: hydroxy, amino, carbonyl, C₁₋₆ alkyl, C₂₋₆ alkenyl, C₂₋₆ alkynyl, and C₁₋₆ alkoxy;
R₄ is absent or is H, or is selected from the following structures:

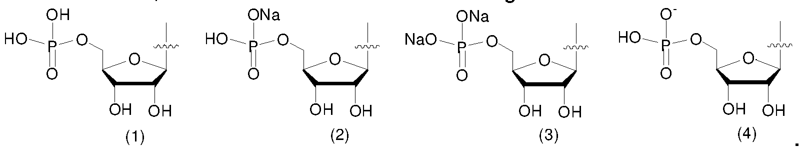

In certain embodiments, 1, 2 or 3 of X₁, X₂, X₃ and X₄ is N.

In certain embodiments, 1 or 2 of X₁, X₂, X₃ and X₄ is N.

In certain embodiments, X₁ is N. In certain embodiments, X₁ is C. In certain embodiments, X₂ is C. In certain embodiments, X₃ is C. In certain embodiments, X₃ is N. In certain embodiments, X₄ is N. In certain embodiments, X₄ is S.

In certain embodiments, m is 1. In certain embodiments, m is 2.

In certain embodiments, R₁ is absent. In certain embodiments, R₁ is H. In certain embodiments, R₁ is C₁₋₆ alkyl.

In certain embodiments, R₂ is absent. In certain embodiments, R₂ is selected from - (CH₂)ₙ-COOH or -(CH₂)ₙ-CONH₂. In certain embodiments, n is 0, 1 or 2. In certain embodiments, the -(CH₂)ₙ- is substituted by amino. In certain embodiments, the amino is substituted by -(C=O)-(CH₂)_{q}-NH₂. In certain embodiments, q is 2.

In certain embodiments, R₃ is absent. In certain embodiments, R₃ is H.

In certain embodiments, R₄ is absent. In certain embodiments, R₄ is H.

In certain embodiments, R₄ has one of the structures shown in the formulas (1), (2), (3) and (4).

In certain embodiments, the compound shown in the formula (I) has one of the following structures:

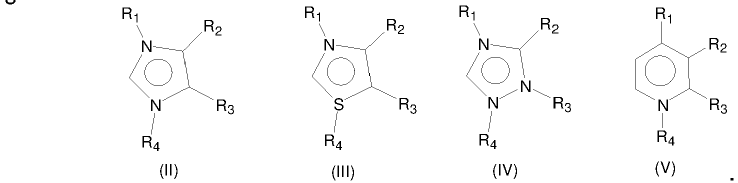

In certain embodiments, the compound shown in the formula (I) has the structure shown in formula (II), (III) or (V).

In certain embodiments, R₂ is selected from -(CH₂)ₙ-COOH or -(CH₂)ₙ-CONH₂; n is 0, 1 or 2; optionally, -(CH₂)ₙ- is substituted by amino; optionally, the amino is substituted by -(C=O)-(CH₂)_{q}-NH₂; preferably, q is 0, 1 or 2. In certain embodiments, R₄ is absent.

In certain embodiments, the compound shown in the formula (I) has one of the structures shown in the formulas (II), (III), (IV) and (V); R₂ and R₃ form a 6-membered heteroaryl group (e.g. pyrimidinyl) comprising 1 or 2 nitrogen atoms; the heteroaryl is optionally substituted by 1 or 2 substituents, and the substituents each are independently selected from: hydroxy, amino, carbonyl, C₁₋₆ alkyl, C₂₋₆ alkenyl, C₂₋₆ alkynyl, and C₁₋₆ alkoxy.

In certain embodiments, the compound shown in the formula (I) has one of the following structures: 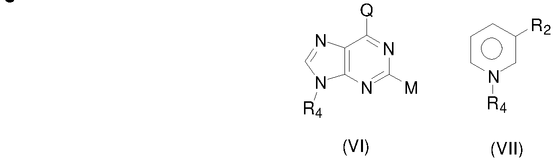 wherein, Q and M each are independently selected from: hydrogen, hydroxy, amino, carbonyl, C₁₋₆ alkyl, C₂₋₆ alkenyl, C₂₋₆ alkynyl, and C₁₋₆ alkoxy. In certain embodiments, Q and M each are independently selected from hydrogen, hydroxy, amino, and carbonyl.

In certain embodiments, Q is selected from: hydrogen, amino, hydroxy, and carbonyl. In certain embodiments, Q is amino.

In certain embodiments, M is selected from: hydrogen and amino. In certain embodiments, M is hydrogen.

In certain embodiments, R₄ is absent, or, R₄ has one of the structures shown in the formulas (1), (2), (3) and (4). In certain embodiments, R₄ is absent or has the structure shown in the formula (2).

In certain embodiments, the compound shown in the formula (I) includes but is not limited to the following compounds:

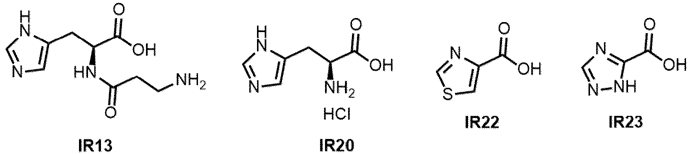

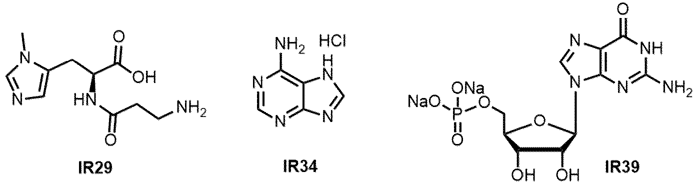

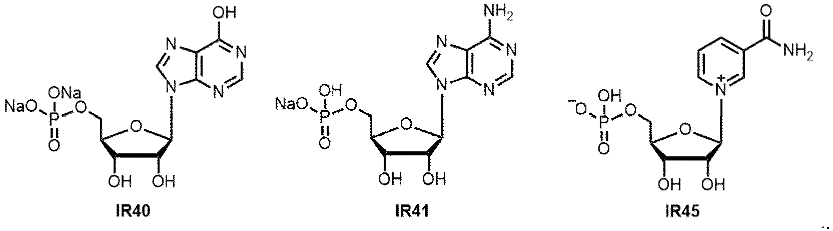

In certain embodiments, the compound shown in the formula (I) is selected from the following compounds:

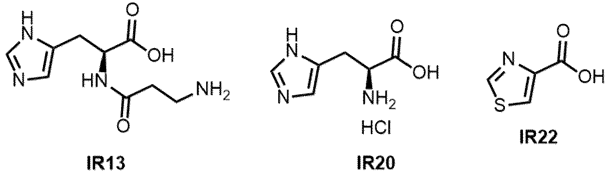

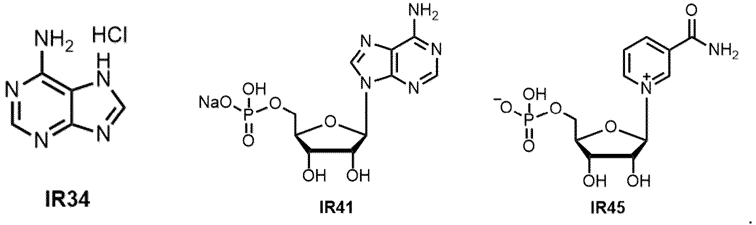

In certain embodiments, the compound shown in the formula (I) is selected from the following compounds:

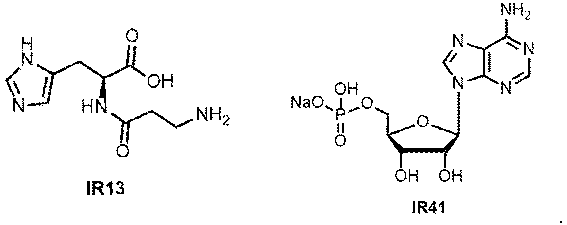

In certain embodiments, the compound shown in the formula (I) is adenosine 5'-monophosphate monosodium salt (IR41).

The term "alkyl" as used herein includes saturated straight or branched monovalent hydrocarbon group having 1 to 6 carbon atoms, or 1 to 4 carbon atoms, or 1 to 3 carbon atoms, or 1 to 2 carbon atoms, wherein the alkyl group may be independently and optionally substituted by one or more substituents described herein. Further examples of the alkyl group include, but are not limited to, methyl (Me, -CH₃), ethyl (Et, -CH₂CH₃), n-propyl (*n*-Pr, -CH₂CH₂CH₃), iso-propyl (*i*-Pr, -CH(CH₃)₂), n-butyl (*n*-Bu, -CH₂CH₂CH₂CH₃), iso-butyl (*i*-Bu, -CH₂CH(CH₃)₂), sec-butyl (*s*-Bu, -CH(CH₃)CH₂CH₃), tert-butyl (*t*-Bu, - C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃) (CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃) (CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃).

The term "alkenyl" denotes a straight or branched monovalent hydrocarbon group having 2 to 6 carbon atoms, or 2 to 4 carbon atoms, and at least one position of the group is unsaturated, i.e., at least one carbon-carbon is an sp2 double bond, wherein the alkenyl group may be independently and optionally substituted by one or more substituents described herein, including the positioning of the group as "trans", "cis" or "E", "Z". Specific examples of the alkenyl group include, but are not limited to, vinyl (-CH=CH₂), allyl (-CH₂CH=CH₂), and 3-buten-1-yl (-CH₂CH₂CH=CH₂), and the like.

The term "alkynyl" denotes a straight or branched monovalent hydrocarbon group having 2 to 6 carbon atoms, or 2 to 4 carbon atoms, and at least one position of the group is unsaturated, i.e., at least one carbon-carbon is a sp triple bond, wherein the alkynyl group may be independently and optionally substituted by one or more substituents described herein. Specific examples of the alkynyl group include, but are not limited to, ethynyl (-C≡CH) and propargyl (-CH₂C≡CH).

The term "alkoxy" as used herein refers to an alkyl group, as defined herein, attached to the rest of a compound molecule through an oxygen atom; in certain examples, the alkoxy group is C₁₋₄ alkoxy; such examples include, but are not limited to, methoxy, ethoxy, propoxy, butoxy and the like. And, the alkoxy group may be independently unsubstituted or substituted by one or more substituents described herein.

The term "heteroaryl" denotes monocyclic, bicyclic and tricyclic ring systems comprising 5 to 12 ring atoms, or 5 to 10 ring atoms, or 5 to 6 ring atoms, wherein at least one ring system is aromatic and at least one ring system comprises one or more heteroatoms, wherein each ring includes 5 to 7 atoms with one or more attaching points linked to the rest of the molecule. The term "heteroaryl" may be used interchangeably with the term "heteroaromatic ring" or "heteroaromatic ring compound". The heteroaryl group is optionally substituted by one or more substituents described herein. In one embodiment, a heteroaryl group consisting of 5 to 10 atoms comprises 1, 2, 3 or 4 heteroatoms independently selected from O, S and N, wherein the nitrogen atom may be further oxidized.

Examples of the heteroaryl group include, but are not limited to: furyl, imidazolyl (e.g., N-imidazolyl, 2-imidazolyl, 4-imidazolyl, 5-imidazolyl), isoxazolyl, oxazolyl (e.g., 2-oxazolyl, 4-oxazolyl, 5-oxazolyl), pyrrolyl (e.g., *N*-pyrrolyl, 2-pyrrolyl, 3-pyrrolyl), pyridyl, pyrimidinyl (e.g., 2-pyrimidinyl, 4-pyrimidinyl, 5-pyrimidinyl), pyridazinyl, thiazolyl (e.g., 2-thiazolyl, 4-thiazolyl, 5-thiazolyl), tetrazolyl (e.g., 5-tetrazolyl), triazolyl, thienyl (e.g., 2-thienyl, 3-thienyl), pyrazolyl, isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl; examples also include, but are by no means limited to the following bicyclic rings: benzimidazolyl, benzofuryl, benzothienyl, indolyl (e.g., 2-indolyl), purinyl, quinolinyl (e.g., 2-quinolinyl, 3-quinolinyl, 4-quinolinyl), 1,2,3,4-tetrahydroisoquinolinyl, 1,3-benzodioxolyl, indolinyl, isoquinolinyl (e.g., 1-isoquinolinyl, 3-isoquinolinyl, or 4-isoquinolinyl), imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridyl and the like.

The term "isomer" includes all isomeric forms (e.g., enantiomers, diastereomers, geometric isomers or conformational isomers), such as R, S configurations containing an asymmetric center, (Z), (E) isomers containing double bonds, and conformational isomers of (Z), (E). Thus, individual stereochemical isomers of the compounds of the invention or mixtures of enantiomers, diastereomers, geometric isomers or conformational isomers thereof are all within the scope of the invention.

The term "salt" includes organic and inorganic salts of the compounds used in the invention. Salts formed from acids include, but are not limited to: inorganic acid salts formed by reaction with amino groups, such as hydrochloride, hydrobromide, phosphate, sulfate, perchlorate; organic acid salts such as acetate, oxalate, maleate, tartrate, citrate, succinate, malonate; or these salts obtained by other methods described in the literature, such as ion exchange. Other acceptable salts include adipate, malate, 2-hydroxypropionate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, glucoheptonate, glycerophosphate, gluconate, hemisulfate, heptanoate, hexanoate, hydroiodiate, 2-hydroxyethanesulfonate, lactobionate, lactate, laurate, lauryl sulfate, malate, methanesulfonate, 2-naphthalenesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, tervalerate and the like. Salts obtained with suitable bases include alkali metal, alkaline earth metal, ammonium and N⁺(C₁₋₄ alkyl)₄ salts. The invention also contemplates quaternary ammonium salts formed by any compound having N-containing group. Water-soluble or oil-soluble or dispersion products can be obtained by quaternization. Alkali metal or alkaline earth metal that can form salts includes sodium, lithium, potassium, calcium, magnesium and the like. Acceptable salts further include suitable, non-toxic amine cations formed by ammonium or quaternary ammonium salts and counterions, such as halides, hydroxides, carboxylates, sulfates, phosphates, nitrates, C₁₋₈ sulfonates and aromatic sulfonates.

The term "solvate" refers to an association formed by one or more solvent molecules and the compound of the invention. The solvent that forms the solvate includes, but is not limited to: water, isopropanol, ethanol, methanol, dimethyl sulfoxide, ethyl acetate, acetic acid and aminoethanol.

The term "hydrate" refers to an association formed by the solvent molecule being water.

The term "N-oxide" refers to an *N*-oxide formed by oxidizing one or more nitrogen atoms of a compound that comprises several amine functional groups. Special examples of N-oxides include N-oxides of tertiary amines or N-oxides formed by nitrogen atoms of nitrogen-containing heterocyclic ring. A corresponding amine can be treated with an oxidizing agent such as hydrogen peroxide or a peracid (e.g., peroxycarboxylic acid) to form the N-oxide (see Advanced Organic Chemistry, Wiley Interscience, 4th Edition, Jerry March, pages). In particular, the N-oxide may be prepared by the method of L. W. Deady (Syn. Comm. 1977, 7, 509-514), for example by reacting an amine compound with m-chloroperoxybenzoic acid (MCPBA) in an inert solvent (such as methylene chloride).

In certain embodiments, the nucleic acid protective agent is used to protect nucleic acids, reduce or avoid photodamage (light-induced damage) or oxidative damage.

In certain embodiments, the nucleic acid detection involves detecting a fluorescence signal. In certain embodiments, the fluorescence signal can be generated by a light reaction or a non-light reaction (e.g., a bioluminescent reaction).

In certain embodiments, the bioluminescent reaction is a reaction in which luciferase catalyzes its substrate to generate a fluorescence signal. The term "light reaction" as used herein refers to a reaction that is exposed to an optical energy source. Typically, in such reaction, an optical energy source is provided to observe the production and/or consumption of a reactant or product having a particular optical characteristic indicative of its presence, for example a change in the absorption spectrum and/or emission spectrum (change in intensity, wavelength and the like) of the reaction mixture or a component thereof.

The term "non-light reaction" as used herein refers to a reaction that occurs without the aid of an optical energy source. In non-light reaction, an optical signal can be generated by means such as bioluminescence, whereby the production and/or consumption of a reactant or product can be observed.

The term "bioluminescence" as used herein refers to the phenomenon of light emission by means of energy generated by natural enzymatic chemical reactions, which belongs to chemiluminescence. Bioluminescence occurs naturally in many organisms, such as fireflies, jellyfish in the deep sea, and some bacteria.

In certain embodiments, the light reaction includes the generation of an optical signal (e.g., a fluorescence signal) that is caused after base extension. In certain embodiments, the optical characteristic in the light reaction described herein is preferably fluorescence.

In certain embodiments, the nucleic acid detection is nucleic acid sequencing or other scenarios (e.g., quantitative PCR).

In certain embodiments, the nucleic acid detection is sequencing, such as high-throughput sequencing, further such as sequencing by synthesis (SBS sequencing), ligation sequencing, hybridization sequencing, nanopore sequencing, or combinatorial probe-anchor ligation (cPAL) sequencing.

In certain embodiments, the nucleic acid detection is quantitative PCR.

### Reagent, kit and use thereof

In one aspect, the invention provides a reagent comprising one or more heteroaromatic ring compounds as defined above, and further comprising a Tris buffer solution.

In the invention, the Tris buffer solution refers to a buffer solution comprising tri(hydroxymethyl)aminomethane (Tris) as a buffer system.

Tris is widely used in preparation of buffer solutions for biochemistry and molecular biology. Tris is a weak base, the aqueous solution of the base has a pH of about 10.5, and hydrochloric acid is added to adjust the pH to the required value, thus obtaining the buffer solution with the pH value. Tris and its hydrochloride (Tris-HCl) may also be used to prepare buffer solutions.

The effective buffering range of the Tris buffer solution is between pH7.0 and pH9.2. The Tris buffer solution may be used as a solvent for dissolving nucleic acid.

In certain embodiments, the heteroaromatic ring compound in the reagent has a concentration of 0.1-500 mM, e.g., 0.1-0.5 mM, 0.5-1 mM, 1-5 mM, 5-10 mM, 10-50 mM, 50-100 mM, 100-200 mM, or 200-500 mM.

In certain embodiments, the reagent is a scanning reagent. Preferably, the heteroaromatic ring compound acts as a nucleic acid protective agent in nucleic acid sequencing.

In certain embodiments, the reagent further comprises sodium chloride and/or a stabilizer for DNA (e.g., Tween-20). The role of sodium chloride is to provide a salt solution background to protect the primer in the test.

In another aspect, the invention provides a kit comprising a reagent of the invention.

In certain embodiments, the kit of the invention may further comprise one or more additional reagents required for nucleic acid detection, e.g., a primer, a polymerase, a buffer solution, a wash solution, or any combination thereof.

In certain embodiments, the kit of the invention is used for sequencing.

In certain embodiments, the kit of the invention may further comprise: a reagent for immobilizing (e.g., by covalent or non-covalent ligation) nucleic acid molecules to be sequenced to a support; a primer for initiating a nucleotide polymerization reaction; a polymerase for performing a nucleotide polymerization reaction; one or more buffer solutions; one or more wash solutions; or any combination thereof.

In certain embodiments, the kit of the invention may further comprise a reagent and/or device for extracting nucleic acid molecules from a sample. Methods for extracting nucleic acid molecules from a sample are well known in the art. Thus, various reagents and/or devices for extracting nucleic acid molecules, such as reagents for disrupting cells, reagents for precipitating DNA, reagents for washing DNA, reagents for solubilizing DNA, reagents for precipitating RNA, reagents for washing RNA, reagents for solubilizing RNA, reagents for removing proteins, reagents for removing DNA (e.g., when the nucleic acid molecule of interest is RNA), reagents for removing RNA (e.g., when the nucleic acid molecule of interest is DNA), and any combination thereof, may be configured in the kit of the invention as desired.

In certain embodiments, the kit of the invention further comprises a reagent for pretreating nucleic acid molecules. In the kit of the invention, the reagent for pretreating nucleic acid molecules is not additionally limited and may be selected according to actual needs. The reagent for pretreating nucleic acid molecules includes, for example, a reagent (e.g., DNase I) for fragmenting nucleic acid molecules, a reagent (e.g., DNA polymerase such as T4 DNA polymerase, Pfu DNA polymerase, Klenow DNA polymerase) for filling up the ends of nucleic acid molecules, an adapter molecule, a tag molecule, a reagent for attaching an adapter molecule to a nucleic acid molecule of interest (e.g., ligase such as T4 DNA ligase), a reagent for repairing the end of nucleic acid (e.g., DNA polymerase that loses 3'-5' exonuclease activity but exhibits 5'-3' exonuclease activity), a reagent for amplifying nucleic acid molecules (e.g., DNA polymerase, primer, dNTP), a reagent (e.g., chromatography column) for isolating and purifying nucleic acid molecules, and any combination thereof.

In certain embodiments, the kit of the invention further comprises a support for immobilizing the nucleic acid molecule to be sequenced. In general, the support used to immobilize the nucleic acid molecule to be sequenced is in a solid phase to facilitate handling. Thus, in the present disclosure, the "support" is also sometimes referred to as "solid support" or "solid phase support". However, it should be understood that the "support" mentioned herein is not limited to a solid, and it may also be a semi-solid (e.g., a gel).

As used herein, the terms "loading", "immobilizing" and "attaching", when used in reference to a nucleic acid, mean directly or indirectly attaching to a solid support via a covalent or non-covalent bond. In certain embodiments of the present disclosure, the method of the invention comprises immobilizing a nucleic acid on a solid support via covalent attachment. However, in general, it is only required that a nucleic acid remains immobilized or attached to a solid support under conditions in which use of the solid support is desired (e.g., in applications in which nucleic acid amplification and/or sequencing is desired). In certain embodiments, immobilizing a nucleic acid on a solid support may comprise immobilizing an oligonucleotide to be used as a capture primer or an amplification primer on the solid support such that the 3' end is available for enzymatic extension, and at least a portion of the primer sequence is capable of hybridizing to a complementary nucleic acid sequence; the nucleic acid to be immobilized is then hybridized to the oligonucleotide, in which case the immobilized oligonucleotide or polynucleotide may be in the 3'-5' direction. In certain embodiments, immobilizing a nucleic acid on a solid support may comprise binding a nucleic acid binding protein to the solid support by an amination modification, and capturing nucleic acid molecules by the nucleic acid binding protein. Alternatively, loading may occur by other means than base pairing hybridization, such as covalent attachment as described above. Non-limiting examples of the means of attaching a nucleic acid to a solid support include nucleic acid hybridization, biotin streptavidin binding, sulfhydryl binding, photoactivated binding, covalent binding, antibody-antigen, physical restriction via hydrogel or other porous polymer, and the like. Various exemplary methods for immobilizing a nucleic acid on a solid support can be found, for example, in G. Steinberg-Tatman et al, Bioconjugate Chemistry 2006, 17, 841-848; Xu X. et al, Journal of the American Chemical Society 128(2006) 9286-9287; US patent applications US 5639603, US 5641658, US 2010248991; international patent applications WO 2001062982, WO 2001012862, WO 2007111937, WO0006770, all of which are incorporated herein by reference in their entirety for all purposes, particularly for all teachings associated with the preparation of a solid support having a nucleic acid immobilized thereon.

In the invention, the support may be made of various suitable materials. Such materials include, for example: minerals, natural polymers, synthetic polymers, and any combination thereof. Specific examples include, but are not limited to: cellulose, cellulose derivatives (e.g. nitrocellulose), acrylics, glass, silica gel, silica, polystyrene, gelatin, polyvinylpyrrolidone, copolymers of vinyl and acrylamide, polystyrene crosslinked with divinylbenzene and the like (see, e.g., Merrifield Biochemistry 1964, 3, 1385-1390), polyacrylamide, latex, dextran, rubber, silicon, plastic, natural sponge, metal plastic, cross-linked dextran (e.g., Sephadex^{™}), agarose gel (Sepharose^{™}), and other supports known to those skilled in the art.

In certain preferred embodiments, the support used to immobilize nucleic acid molecules to be sequenced may be a solid support comprising an inert substrate or matrix (e.g., glass slide, polymer beads and the like) that has been functionalized, for example, by application of an intermediate material comprising reactive groups that allow covalent attachment of biomolecules such as polynucleotides. Examples of such support include, but are not limited to, polyacrylamide hydrogels supported on inert substrates such as glass, in particular polyacrylamide hydrogels as described in WO 2005/065814 and US 2008/0280773, the contents of which are incorporated herein by reference in their entirety. In such embodiments, a biomolecule (e.g., polynucleotide) can be directly covalently attached to the intermediate material (e.g., hydrogel), while the intermediate material itself can be non-covalently attached to a substrate or matrix (e.g., glass substrate). In certain preferred embodiments, the support is a glass slide or silicon wafer modified with a layer of avidin, amino, acrylamide silane or aldehyde chemical groups on the surface.

In the invention, the support or solid support is not limited in its size, shape and configuration. In certain embodiments, the support or solid support is a planar structure, such as a slide, chip, microchip and/or array. The surface of such a support may be in the form of a planar layer.

In certain preferred embodiments, the support used to immobilize nucleic acid molecules to be sequenced is an array of beads or wells (which is also referred to as a chip). The array may be prepared using any of the materials outlined herein for preparing solid supports, and preferably the surfaces of the beads or wells on the array are functionalized to facilitate the immobilization of nucleic acid molecules. The number of beads or wells on the array is not limited. For example, each array may comprise 10-10², 10²-10³, 10³-10⁴, 10⁴-10⁵, 10⁵-10⁶, 10⁶-10⁷, 10⁷-10⁸, 10⁸-10⁹, 10¹⁰-10¹¹, 10¹¹-10¹² or more beads or wells. In certain exemplary embodiments, one or more nucleic acid molecules can be immobilized on the surface of each bead or well. Accordingly, 10-10², 10²-10³, 10³-10⁴, 10⁴-10⁵, 10⁵-10⁶, 10⁶-10⁷, 10⁷-10⁸, 10⁸-10⁹, 10¹⁰-10¹¹, 10¹¹-10¹² or more nucleic acid molecules can be immobilized per array. Such arrays may therefore be particularly advantageous for high-throughput sequencing of nucleic acid molecules.

In certain preferred embodiments, the kit of the invention further comprises a reagent for immobilizing (e.g., by covalent or non-covalent ligation) a nucleic acid molecule to be sequenced to a support. Such reagent includes, for example, a reagent for activating or modifying the nucleic acid molecule (e.g., at its 5' end), such as phosphate, thiol, amine, carboxylic acid or aldehyde; a reagent for activating or modifying the surface of the support, such as amino-alkoxysilane (e.g., aminopropyltrimethoxysilane, aminopropyltriethoxysilane, 4-aminobutyltriethoxysilane and the like); crosslinkers, such as succinic anhydride, phenyl diisothiocyanate (Guo et al, 1994), maleic anhydride (Yang et al, 1998), 1-ethyl-3-(3-dimethylaminopropyl)-carbodiimide hydrochloride (EDC), m-maleimidobenzoic acid-N-hydroxysuccinimide ester (MBS), N-succinimidyl [4-iodoacetyl] aminobenzoic acid (SIAB), N-succinimidyl 4-(maleimidomethyl)cyclo-hexanecarboxylate (SMCC), N-γ-maleimidobutyryloxy-succinimide ester (GMBS), succinimidyl 4-(p-maleimidophenyl)butyrate (SMPB); and any combination thereof.

In certain preferred embodiments, the kit of the invention further comprises a primer for initiating nucleotide polymerization reaction. In the invention, the primer is not particularly limited as long as it can be specifically annealed to a region of the target nucleic acid molecule. In certain exemplary embodiments, the length of the primer may be 5-50bp, such as 5-10, 10-15, 15-20, 20-25, 25-30, 30-35, 35-40, 40-45, 45-50 bp. In certain exemplary embodiments, the primer may comprise naturally occurring or non-naturally occurring nucleotides. In certain exemplary embodiments, the primer comprises or consists of naturally occurring nucleotides. In certain exemplary embodiments, the primer comprises modified nucleotides, such as Locked Nucleic Acid (LNA). In certain preferred embodiments, the primer comprises a universal primer sequence.

In certain preferred embodiments, the kit of the invention further comprises a polymerase for performing a nucleotide polymerization reaction. In the invention, polymerization reaction can be carried out using various suitable polymerases. In certain exemplary embodiments, the polymerase (e.g., DNA polymerase) is capable of synthesizing a new DNA strand using DNA as a template. In certain exemplary embodiments, the polymerase (e.g., reverse transcriptase) is capable of synthesizing a new DNA strand using RNA as a template. In certain exemplary embodiments, the polymerase (e.g., RNA polymerase) is capable of synthesizing a new RNA strand using DNA or RNA as a template. Thus, in certain preferred embodiments, the polymerase is selected from DNA polymerase, RNA polymerase, and reverse transcriptase.

In certain preferred embodiments, the kit of the invention further comprises one or more cleavage reagents.

The term "cleavage reagent" used herein refers to a reagent which, during nucleic acid detection, is capable of removing blocking groups and/or detectable labels from nucleic acid intermediates (i.e., growing nucleic acid strands) to expose the ribose 3'OH end, which can be used for the next cycle of test. In certain embodiments, the cleavage reagent is tris(3-hydroxypropyl) phosphine (THPP), which is useful for cleaving disulfide-containing blocking groups. In certain embodiments, the cleavage reagent is a thiol reagent, such as dimercaprol, which can be used to cleave azide-containing blocking groups. In certain embodiments, the cleavage reagent is tris(2-carboxyethyl) phosphine (TCEP), which can be used to cleave fluorophore-containing detectable labels.

In certain preferred embodiments, the kit of the invention further comprises one or more buffer solutions. Such buffer solution includes, but is not limited to, a buffer solution for DNase I, a buffer solution for DNA polymerase, a buffer solution for ligase, a buffer solution for eluting nucleic acid molecules, a buffer solution for solubilizing nucleic acid molecules, a buffer solution for performing a nucleotide polymerization reaction (e.g., PCR), and a buffer solution for performing a ligation reaction. The kit of the invention may comprise any one or more of the above-described buffer solutions.

In certain embodiments, the buffer solution for DNA polymerase comprises monovalent salt ions (e.g., sodium ions, chloride ions) and/or divalent salt ions (e.g., magnesium ions, sulfate ions, manganese ions). In certain embodiments, the concentration of the monovalent or divalent salt ions in the buffer solution is 10 µM to 200 mM, e.g., 10 µM, 50 µM, 100 µM, 200 µM, 500 µM, 1 mM, 3 mM, 10 mM, 20 mM, 50 mM, 100 mM, 150 mM, or 200 mM.

In certain embodiments, the buffered solution for DNA polymerase comprises tri(hydroxymethyl)aminomethane (Tris). In certain embodiments, the concentration of Tris in the buffer solution is 10 mM-200 mM, e.g., 10 mM, 20 mM, 50 mM, 100 mM, 150 mM, or 200 mM.

In certain embodiments, the buffer solution for DNA polymerase comprises an organic solvent, such as DMSO or glycerol. In certain embodiments, the organic solvent is present in the buffer solution in an amount of 0.01% to 10%, e.g., 0.01%, 0.02%, 0.05%, 1%, 2%, 5%, or 10%, by mass.

In certain embodiments, the pH of the buffer solution for DNA polymerase is 7.0-9.0, e.g., 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0.

In certain embodiments, the buffer solution for DNA polymerase comprises: monovalent salt ions (e.g., sodium ions, chloride ions), divalent salt ions (e.g., magnesium ions, sulfate ions, manganese ions), Tris and an organic solvent (e.g., DMSO or glycerol). In certain embodiments, the pH of the buffer solution is 8.8.

In certain preferred embodiments, the kit of the invention further comprises one or more wash solutions. Examples of such wash solutions include, but are not limited to, phosphate buffer, citrate buffer, Tris-HCl buffer, acetate buffer, carbonate buffer and the like. The kit of the invention may comprise any one or more of the above-described wash solutions.

In another aspect, the invention provides use of a reagent or kit of the invention for nucleic acid detection.

In certain embodiments, the nucleic acid detection involves detecting a fluorescence signal.

In certain embodiments, the fluorescence signal can be generated by a light reaction or a non-light reaction (e.g., a bioluminescent reaction).

In certain embodiments, the bioluminescence refers to a reaction in which a luciferase catalyzes its substrate to generate a fluorescence signal.

In certain embodiments, the nucleic acid detection involves a light reaction or a non-light reaction (e.g., a bioluminescent reaction).

In certain embodiments, the light reaction includes the generation of an optical signal (e.g., a fluorescence signal) that is caused after base extension.

In certain embodiments, the optical characteristic in the light reaction of the invention is preferably fluorescence.

In certain embodiments, the nucleic acid detection may be used for nucleic acid sequencing or other scenarios (e.g., quantitative PCR).

In certain embodiments, the nucleic acid detection is sequencing, such as high-throughput sequencing, further such as sequencing by synthesis (SBS), ligation sequencing, hybridization sequencing, nanopore sequencing, or cPAL sequencing.

In certain embodiments, the nucleic acid detection is quantitative PCR.

In another aspect, the invention provides a method of preparing the reagent of the invention, comprising dissolving various components of the reagent in ultrapure water to produce a clear, homogeneous solution, and then filtering the solution to obtain the reagent of the invention. Ultrasound-assisted dissolution may be used.

### Method for inhibiting degradation of nucleic acids and method for detecting a target nucleic acid molecule

In another aspect, the invention provides a method for inhibiting degradation of a nucleic acid in nucleic acid detection, which comprises, when detecting the nucleic acid to be tested in a reaction mixture, adding one or more heteroaromatic ring compounds as defined above to inhibit degradation of the nucleic acid to be tested.

In certain embodiments, the test involves detecting a fluorescence signal, and the reaction mixture comprises a reactant that generates a fluorescence signal, a nucleic acid to be tested, and a buffer.

In certain embodiments, the reactant that generates a fluorescence signal comprises: a labeled or unlabeled nucleotide (e.g., dNTP), and optionally further comprises another reagent that allows the reactant to generate a fluorescence signal, such as a polymerase, a primer, and/or a fluorescent probe.

In certain embodiments, the fluorescence signal is generated by a light reaction or a non-light reaction (e.g., bioluminescence).

In another aspect, the invention provides a method for detecting a target nucleic acid molecule, which comprises detecting the target nucleic acid molecule in a reaction mixture in the presence of one or more heteroaromatic ring compounds as defined above.

In certain embodiments, the detection involves detecting a fluorescence signal, and the reaction mixture comprises a reactant that generates a fluorescence signal, a target nucleic acid molecule, and a buffer.

In certain embodiments, the reactant that generates a fluorescence signal comprises: a labeled or unlabeled nucleotide (e.g., dNTP), and optionally further comprises another reagent that allows the reactant to generate a fluorescence signal, such as a polymerase, a primer, and/or a fluorescent probe.

In certain embodiments, the fluorescence signal is generated by a light reaction or a non-light reaction (e.g., bioluminescence).

In certain embodiments, the method is sequencing or quantitative PCR.

In certain embodiments, the sequencing is SBS sequencing, ligation sequencing, hybridization sequencing, nanopore sequencing, or cPAL sequencing.

In another aspect, the invention provides a method for determining a target single-stranded polynucleotide sequence, comprising:
(a) incorporating one or more nucleotides on a complementary strand of the target single-stranded polynucleotide sequence;
(b) providing a condition for an incorporated nucleotide to generate a fluorescence signal in the presence of one or more heteroaromatic ring compounds as defined above and detecting the fluorescence signal to determine type of the incorporated nucleotide.

In certain embodiments, the method further comprises, (c) repeating steps (a) - (b) at least once.

In certain embodiments, the method further comprises, (d) determining the target single-stranded polynucleotide sequence.

In certain embodiments, the heteroaromatic ring compound has a concentration of greater than 0.1 mM, preferably 0.1-500 mM (e.g., 0.1-0.5 mM, 0.5-1 mM, 1-5 mM, 5-10 mM, 10-50 mM, 50-100 mM, 100-200 mM, or 200-500 mM), more preferably 5-20 mM, and most preferably 10 mM. In the invention, the incorporated nucleotide is a natural or modified nucleotide analog. Nucleotide typically comprises sugar, nucleobase, and at least one phosphate group. Nucleotide includes deoxyribonucleotide, modified deoxyribonucleotide, ribonucleotide, modified ribonucleotide, peptide nucleotide, modified peptide nucleotide, modified phosphate sugar backbone nucleotide, and mixtures thereof. Examples of nucleotide include, for example, adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP). Nucleotide analogs comprising modified nucleobases can also be used in the method described herein. Exemplary modified nucleobases that can be comprised in a polynucleotide, whether having a natural backbone or a similar structure, include, for example, inosine, xanthine, hypoxanthine, isocytosine, isoguanine, 2-aminopurine, 5-methylcytosine, 5-hydroxymethylcytosine, 2-aminoadenine, 6-methyladenine, 6-methylguanine, 2-propylguanine, 2-propyladenine, 2-thiouracil, 2-thiothymine, 2-thiocytosine, 15-halouracil, 15-halocytosine, 5-propynyluracil, 5-propynylcytosine, 6-azouracil, 6-azocytosine, 6-azothymine, 5-uracil, 4-thiouracil, 8-halo adenine or guanine, 8-amino adenine or guanine, 8-thio adenine or guanine, 8-sulfanyl adenine or guanine, 8-hydroxy adenine or guanine, 5-halo substituted uracil or cytosine, 7-methyl guanine, 7-methyl adenine, 8-aza guanine, 8-aza adenine, 7-deazaguanine, 7-deazaadenine, 3-deazaguanine, 3-deazaadenine and the like.

In certain embodiments, the incorporated nucleotide includes a sugar unit, a nucleobase, a phosphate group, and a cleavable blocking group (e.g., a 3' blocking group) to reversibly prevent further extension of the complementary strand of the target single-stranded polynucleotide sequence.

In certain embodiments, the nucleobase is selected from adenine (A), guanine (G), thymine (T), cytosine (C) and uracil (U).

In certain embodiments, each type of nucleotide is capable of generating a fluorescence signal that is distinguishable from each other to identify the type of the incorporated nucleotide. For example, four deoxyribonucleotides comprising adenine (A), guanine (G), cytosine (C), and thymine (T), respectively, can produce different fluorescence signals, making them readily distinguishable from each other.

In certain embodiments, the fluorescence signal is generated by a light reaction or a bioluminescent reaction.

In certain embodiments, the method further comprises, between (b) and (c), (b') removing from the incorporated nucleotide a moiety capable of generating a fluorescence signal directly or indirectly attached thereto; and/or, washing to remove an unincorporated nucleotide.

In certain embodiments, the method further comprises, between (b) and (c), (b") removing from the incorporated nucleotide a cleavable blocking group, to allow further extension of the complementary strand of the target single-stranded polynucleotide sequence.

In certain embodiments, the target single-stranded polynucleotide is present in a nucleic acid array.

In certain embodiments, each site on the array comprises multiple copies of a single target single-stranded polynucleotide.

In certain embodiments, the nucleic acid array is immobilized on a solid support (e.g., a chip).

### Sequencing based on light reaction

In certain embodiments, the fluorescence signal is generated by a light reaction.

In certain embodiments, the incorporated nucleotide is a fluorescently labeled nucleotide (e.g., dNTP). In certain embodiments, providing a condition for the incorporated nucleotide to generate a fluorescence signal comprises: irradiating the reaction mixture to generate a fluorescence signal, wherein the reaction mixture comprises a fluorescently labeled nucleotide (e.g., dNTP). In certain embodiments, the fluorescently labeled nucleotide is obtainable by linking a linker to a nucleotide (e.g., its base). The linker may be acid labile, photolabile or comprise a disulfide bond.

In certain embodiments, the method comprises: incorporating one or more fluorescently labeled nucleotides on the complementary strand of the target single-stranded polynucleotide sequence; irradiating the reaction mixture in the presence of one or more heteroaromatic ring compounds as defined above to generate a fluorescence signal and detecting the fluorescence signal to determine the type of the incorporated nucleotide. At this time, the reaction mixture includes a reactant (e.g., a fluorescently labeled nucleotide, a polymerase, a primer and the like) that generates a fluorescence signal, a target single-stranded polynucleotide molecule, and a buffer (e.g., Tris buffer and the like), as will be understood by those skilled in the art.

In certain embodiments, the method further comprises: removing from the incorporated nucleotide the fluorescent label attached thereto; and/or, washing to remove an unincorporated nucleotide. In certain embodiments, the method further comprises: removing from the incorporated nucleotide the cleavable blocking group to allow further extension of the complementary strand of the target single-stranded polynucleotide sequence.

In certain embodiments, providing a condition for the incorporated nucleotide to generate a fluorescence signal comprises: irradiating the reaction mixture to generate a fluorescence signal, wherein the reaction mixture comprises an unlabeled nucleotide (e.g., dNTP) and a fluorescently labeled affinity reagent (e.g., an antibody) that is capable of specifically binding to the unlabeled nucleotide. Such embodiments may be referred to as Cool MPS sequencing, the detailed teachings of which may be found in PCT international application WO2018129214A1. In such embodiments, based on SBS sequencing principle, the fluorescent group is not directly labeled on the incorporated nucleotide, but on an affinity reagent (e.g., antibody, aptamer, Affimer, Knottin and the like) that is capable of specifically binding to a base, sugar, cleavable blocking group, or combination of these components incorporated into the nucleotide, and thus the type of the incorporated nucleotide can be identified by the affinity reagent.

In certain embodiments, the method comprises: incorporating one or more unlabeled nucleotides on the complementary strand of the target single-stranded polynucleotide sequence; providing a fluorescently labeled affinity reagent and indirectly attaching the fluorescent label to the incorporated nucleotide via specific binding between the affinity reagent and the nucleotide; irradiating the reaction mixture in the presence of one or more heteroaromatic ring compounds as defined above to generate a fluorescence signal and detecting the fluorescence signal to determine the type of the incorporated nucleotide.

In certain embodiments, the method further comprises: removing from the incorporated nucleotide the affinity reagent attached thereto; and/or, washing to remove an unincorporated nucleotide. In certain embodiments, the method further comprises: removing from the incorporated nucleotide the cleavable blocking group to allow further extension of the complementary strand of the target single-stranded polynucleotide sequence.

### Sequencing based on bioluminescence reaction

In certain embodiments, the fluorescence signal is generated by a bioluminescent reaction. The detection principle of bioluminescence lies in that the incorporated nucleotide is not directly labeled with a fluorophore, but labeled with an affinity substance such as biotin or digoxin, and after polymerization reaction, a pairing member of the above-mentioned affinity substance is added, where the pairing member carries a luciferase, to indirectly bind the luciferase to the incorporated nucleotide, and then a reaction substrate is added to generate a fluorescence signal, thereby determining the type of the incorporated nucleotide, without the need of excitation light irradiation. Detailed teachings on bioluminescent reaction can be found in, for example, PCT international application WO2020227953A1.

In certain embodiments, the incorporated nucleotide is a nucleotide carrying a tag (e.g., dNTP). In certain embodiments, the tag carried by the nucleotide is any member of molecular pairing capable of specifically binding to each other. Specific binding between pairing members achieves linkage of the nucleotide to the luciferase. Exemplary pairing members include, but are not limited to: (a) a hapten or antigenic compound in combination with a corresponding antibody or binding part or fragment thereof, such as digoxin-digoxin antibody, N3G-N3G antibody, FITC-FITC antibody; (b) nucleic acid aptamers and proteins; (c) non-immunological binding pairs (e.g., biotin-avidin, biotin-streptavidin, biotin-neutral avidin); (d) hormone-hormone binding proteins; (e) receptor-receptor agonists or antagonists; (f) lectin-carbohydrate; (g) enzyme-enzyme cofactors; (h) enzyme-enzyme inhibitors; and (i) complementary oligonucleotide or polynucleotide pairs that can form nucleic acid duplexes.

In certain embodiments, the tag carried by the nucleotide is a small molecule marker selected from biotin, digoxin, N3G or FITC, and the luciferase carries a pairing member corresponding to the small molecule marker. For example, in one specific embodiment, where the tag carried by the nucleotide is biotin, the luciferase may be a streptavidin-labeled luciferase; where the tag carried by the nucleotide is digoxin, the luciferase may be a digoxin antibody-labeled luciferase. The luciferase source includes, but is not limited to, firefly, gaussia, Renilla and other organisms. For example, the streptavidin-labeled luciferase may be SA-Gluc: Streptavidin-Gaussia princeps luciferase from Adivity Co. The digoxin antibody-labeled luciferase may be digoxin antibody-Gluc or digoxin antibody-Nluc.

In certain embodiments, providing a condition for the incorporated nucleotide to generate a fluorescence signal comprises: allowing the reaction mixture to generate a fluorescence signal by a bioluminescent reaction, wherein the reaction mixture comprises a nucleotide carrying a tag (e.g., dNTP), a luciferase capable of specifically binding to the tag and a substrate for the luciferase.

In certain embodiments, the method comprises: incorporating one or more nucleotides carrying a tag on the complementary strand of the target single-stranded polynucleotide sequence; providing a luciferase attached with a pairing member capable of specifically binding to the tag and indirectly attaching the luciferase to the incorporated nucleotide via specific binding between the pairing members; providing a substrate for the luciferase in the presence of one or more heteroaromatic ring compounds as defined above to generate a fluorescence signal and detecting the fluorescence signal to determine the type of the incorporated nucleotide.

In certain embodiments, the method further comprises: removing from the incorporated nucleotide the luciferase attached thereto; and/or, washing to remove an unincorporated nucleotide. In certain embodiments, the method further comprises: removing from the incorporated nucleotide the cleavable blocking group to allow further extension of the complementary strand of the target single-stranded polynucleotide sequence.

The following discussion will be made by way of example of sequencing by synthesis, which is not meant to be limiting. All uses and methods of using the reagent of the invention in the steps of nucleic acid detection involving a light reaction are included within the scope of the invention.

In certain embodiments, the method for determining a target single-stranded polynucleotide sequence comprises:
(a) providing a duplex, a nucleotide, a polymerase and a cleavage reagent; the duplex comprising a growing nucleic acid strand and a nucleic acid molecule to be sequenced;
(b) performing a reaction cycle comprising the following steps (i), (ii) and (iii):
   step (i): incorporating the nucleotide into the growing nucleic acid strand under the action of the polymerase to form a nucleic acid intermediate comprising a blocking group and a detectable label;
   step (ii): detecting the detectable label on the nucleic acid intermediate in the presence of the reagent of the invention; and
   step (iii): removing the blocking group on the nucleic acid intermediate using a cleavage reagent.

In certain embodiments, the reaction cycle further comprises step (iv): removing the detectable label on the nucleic acid intermediate using a cleavage reagent.

In certain embodiments, the method comprises the steps of:
step 1, loading a DNA nanoball (DNB for short) on a prepared sequencing chip;
step 2, pumping a prepared dNTP molecular mixture into the chip, so that the dNTP is added to a complementary strand of the DNA to be tested under the action of a DNA polymerase;
step 3, photographing and scanning, wherein laser is used as excitation wavelength for photographing because the dNTP is modified and carries fluorophore molecules; as the laser has a damage effect on the DNA, a scanning reagent comprising a nucleic acid protective agent is added in the step of photographing, and the nucleic acid protective agent is one or more heteroaromatic ring compounds defined above;
step 4, cleaving the fluorophore at the base end and the blocking group at the ribose 3'OH end on the dNTP using a cleavage reagent, and thoroughly eluting to expose the 3'OH end so as to carry out the next cycle of reaction;
step 5, repeating "step 2, step 3 and step 4" at least once; and
step 6, determining the nucleotide sequence of the nucleic acid molecule to be tested by analyzing the photographing result.

In the method of the invention, the target single-stranded polynucleotide may also be referred to as "target single-stranded polynucleotide molecule", "nucleic acid to be tested", "nucleic acid molecule to be tested" or "target nucleic acid molecule", which are synonymous and interchangeable.

In the method of the invention, the nucleic acid molecule to be tested is not limited in length. In certain preferred embodiments, the nucleic acid molecule to be tested may have a length of at least 10bp, at least 20bp, at least 30bp, at least 40bp, at least 50bp, at least 100bp, at least 200bp, at least 300bp, at least 400bp, at least 500bp, at least 1000bp, or at least 2000 bp. In certain preferred embodiments, the length of the nucleic acid molecule to be tested can be 10-20bp, 20-30bp, 30-40bp, 40-50bp, 50-100bp, 100-200bp, 200-300bp, 300-400bp, 400-500bp, 500-1000bp, 1000-2000bp, or more than 2000 bp. In certain preferred embodiments, the nucleic acid molecule to be tested may have a length of 10-10000bp to facilitate high-throughput sequencing.

In certain preferred embodiments, the nucleic acid molecule to be tested may be pretreated prior to immobilizing it to a support. Such pretreatment includes, but is not limited to, fragmentation of the nucleic acid molecule, filling of the ends, addition of adapters, addition of indexes, amplification of the nucleic acid molecule, isolation and purification of the nucleic acid molecule, and any combination thereof.

The term "nanoball" used herein generally denotes a macromolecule or complex having a compact, e.g., (approximately) spherical shape with an inner diameter typically ranging between about 1 nm and about 1000 nm, preferably between about 50 nm and about 500 nm.

The term "nucleic acid nanoball" used herein is generally a concatemer comprising multiple copies of a target nucleic acid molecule. These copies of nucleic acid are typically disposed one after the other in a continuous linear strand of nucleotides, but the nucleic acid nanoball of the invention can also be prepared from any nucleic acid molecule using the method described herein. This tandem repeat structure, along with the single-stranded nature of the DNA, results in a nanoball folding configuration. In general, the multiple copies of the target nucleic acid molecule in the nucleic acid nanoball each comprise a adapter sequence of known sequence to facilitate amplification or sequencing thereof. The adapter sequences of each target nucleic acid molecule are typically the same, but may also be different. Nucleic acid nanoball generally includes DNA nanoball, also referred to herein as DNB (DNA nanoball).

Nucleic acid nanoball can be generated using, for example, rolling circle replication (RCA). RCA process has been used to prepare multiple sequential copies of M13 genome (Blanco et al, (1989) J Biol Chem 264: 8935-8940). In this method, nucleic acid is replicated in a linear concatemeric manner. Guidance as to the selection of conditions and reagents for RCA reaction can be found by those skilled in the art in a number of references, including U.S. Pat. Nos. US5,426,180, US5,854,033, US6,143,495 and US5,871,921, all of which are incorporated herein by reference in their entirety for all purposes, particularly for all teachings associated with the preparation of nucleic acid nanoballs using RCA or other methods.

The nucleic acid nanoball can be loaded on the surface of the solid support as described herein. The nanoball may be attached to the surface of the solid support by any suitable method. Non-limiting examples of such method include nucleic acid hybridization, biotin streptavidin binding, sulfhydryl binding, photoactivated binding, covalent binding, antibody-antigen, physical restriction via hydrogel or other porous polymer, and the like, or combinations thereof. In some cases, the nanoball may be digested with a nuclease (e.g., a DNA nuclease) in order to generate smaller nanoballs or fragments from the nanoball.

In certain embodiments, the surface of the solid support may carry reactive functional groups that react with the complementary functional groups on the target single-stranded polynucleotide molecules to form covalent bonds, for example, in the same way as the technique used to attach cDNA to a microarray, e.g., see Smirnov et al (2004), Genes, Chromosomes & Cancer, 40: 72-77 and Beaucage (2001), Current Medicinal Chemistry, 8: 1213-1244, both of which are incorporated herein by reference. DNB can also be effectively attached to hydrophobic surfaces, such as clean glass surfaces with low concentrations of various reactive functional groups (e.g., -OH groups). Attachment via covalent bonds formed between the target single-stranded polynucleotide molecule and reactive functional groups on a surface is also referred to herein as "chemical attachment".

In other embodiments, the target single-stranded polynucleotide molecule may be adsorbed onto a surface. In such embodiments, the target single-stranded polynucleotide molecule is immobilized by non-specific interaction with the surface, or by non-covalent interaction such as hydrogen bonding, van der waals forces and the like.

### Advantageous effects

The invention can realize the following beneficial effects:
According to the invention, in nucleic acid detection, the heteroaromatic ring compound is added to bring into contact with a nucleic acid sample, thereby inhibiting the damage and degradation of the nucleic acid sample in the course of treatment and processing. The heteroaromatic ring compound not only has good water solubility, but also has stable chemical property, does not react with oxygen in the air, and is not easy to degrade and deteriorate during storage and use, thereby overcoming the problem of "easy oxidative degradation" in the existing nucleic acid protection strategy.

### Detailed Description of Embodiments

Embodiments of the present application will be described in detail below with reference to the examples, but it will be understood by those skilled in the art that the following examples are only illustrative of the present application and should not be construed as limiting the scope of the present application. The examples, in which specific conditions are not specified, were carried out according to conventional conditions or conditions recommended by the manufacturer. The reagents or equipment used, for which the manufacturer is not indicated, are all conventional products commercially available.

The following description provides more details of the use of the heteroaromatic ring compounds of the invention for massively parallel nucleic acid sequencing. The method can include performing at least 5 cycles of nucleic acid sequencing using a sequencing slide.

Each cycle in the sequencing process includes:
a) incorporating a fluorochrome-labeled nucleotide molecule carrying a cleavable blocking group into a complementary strand of a nucleic acid to be tested;
b) contacting the nucleotide strand with a scanning reagent comprising 0.1mM to 200mM of an additive, wherein the scanning reagent has a pH of about 6.0-9.0;
c) exciting fluorochrome molecules by laser to emit fluorescence and measuring the fluorescence value to obtain the information of the incorporated nucleotide molecules so as to deduce the sequence information of the nucleic acid to be tested;
d) cleaving the fluorochrome group and the cleavable blocking group using a cleavage reagent.

The sequencing slide is loaded with single-stranded DNA nanoballs (DNB), e.g., comprising E. coli genomic DNA. In the examples below, imaging assays are performed using MGI sequencing slides (MGISEQ-2000RS sequencing slides); the kit is MGISEQ2000RS high-throughput (rapid) sequencing reagent kit (FCL PE100) and can be used for PE150 sequencing; the library is standard library reagent V3.0 (26 ng/EA, E320, Barcode 97-104).

### 1. Experimental design

Sequencing system: MGISEQ-2000
Sequencing library: Enterprise reference, Standard library V3.0
Sequencing reagent: HotMPS High-throughput Sequencing Set (G400 HM FCL PE100), Item No: 940-000091-00

Different concentrations of the heteroaromatic ring compound are respectively added into the scanning reagent in the kit, and a control scanning reagent without additive is simultaneously prepared. PE150 sequencings are performed on the same MGISEQ-2000 sequencer, and the sequencing quality by using each scanning reagent is compared after off-line. The data of several scanning reagents, in particular, indices such as second strand Q30 (%), error rate (%) and signal rise value and the like, are compared.

### 2. Evaluation indices and standards

| Evaluation indices | Qualification standards |
|---|---|
| Q30 (%) | with blank control as the standard, the higher the value, the better |
| First strand Q30 (%) | with blank control as the standard, the higher the value, the better |
| Second strand Q30 (%) | with blank control as the standard, the higher the value, the better |
| First strand error rate (%) | with blank control as the standard, the lower the value, the better |
| Second strand error rate (%) | with blank control as the standard, the lower the value, the better |
| Average error rate (%) | with blank control as the standard, the lower the value, the better |
| Signal rise value | with blank control as the standard, the higher the value, the better |

| | |
|---|---|
| Notes: 1. Q30 indicates the proportion of bases with the error rate of Basecall (i.e. base recognition) result lower than 0.001 (accuracy higher than 99.9%); 2. The sensitivity of second strand Q30 (%) is higher than that of Q30 (%) or first strand Q30 (%) as evaluated from the sensitivity. | |

### 3. Experimental procedure

### a. Preparation of DNB

DNB was prepared using a MGISEQ2000RS high-throughput (rapid) sequencing reagent set.
1) DNB preparation buffer, DNB polymerase mixture I, TE buffer and DNB termination buffer were taken out and placed on an ice box for about 0.5 h. After melting, they were shaken with a vortex oscillator for 5 s, then centrifuged briefly and put on the ice box for later use.
2) Using a 0.2 mL 8 - tube strip or PCR tube, a reaction mixture was formulated on ice according to the following system:

| Component | Amount added (µL) |
|---|---|
| Standard library | 3 |
| TE buffer | 17 |
| DNB preparation buffer | 20 |
| Total volume | 40 |

3) The reaction mixture was shaken with the vortex oscillator, centrifuged with a minicentrifuge for 5 s, and placed in a PCR instrument for primer hybridization. The reaction conditions were shown in the following table:

| Temperature | Time |
|---|---|
| Thermal cover (105°C) | On |
| 95°C | 1 min |
| 65°C | 1 min |
| 40°C | 1 min |
| 4°C | Hold |

4) DNB polymerase mixture II (LC) was taken out and placed on an ice box, then centrifuged briefly and put on the ice box for later use.
5) When the temperature of the PCR instrument reached 4°C, the PCR tube was taken out and centrifuged with the minicentrifuge for 5 s, and then the following components were added in condition of ice:

| Component | Amount added to 100 µL system (µL) |
|---|---|
| DNB polymerase mixture I | 40 |
| DNB polymerase mixture II (LC) | 4 |

6) The reaction mixture was shaken with the vortex oscillator, centrifuged with the minicentrifuge for 5 s, and immediately placed in the PCR instrument. The reaction conditions were as follows:

| Temperature | Time |
|---|---|
| Thermal cover (35°C) | On |
| 30°C | 25 min |
| 4°C | Hold |

7) When the temperature of the PCR instrument reached 4°C, the PCR tube was taken out, to which was added 20 µL of the termination buffer, followed by mixing slowly 5-8 times using a wide mouth tip.
8) After the preparation of DNB was completed, 2 µL of the DNB was taken out, and tested for concentration using Qubit^{®} ssDNA Assay Kit and Qubit^{®} Fluorometer instrument.

### b. Preparation of slide and sequencing reagent tank

1) A slide was taken out from a refrigerator at -25°C~-15°C, and placed in a room temperature environment for at least 60 min (no more than 24 h).
2) DNB loading buffer II was taken out, and placed on an ice box for about 0.5 h. After melting, it was shaken with the vortex oscillator for 5 s, then centrifuged briefly and put on the ice box for later use.
3) Using a new 1.5 mL centrifuge tube, a DNB loading system was formulated according to the following table:

| Component | Amount of FCL added (µL) |
|---|---|
| DNB loading buffer II | 33 |
| DNB | 99 |
| Total volume | 132 |

4) The DNB loading system was mixed slowly 5-8 times with a wide mouth tip.
5) MGIDL-200H portable sampler was taken out, and a gasket was installed in a gasket tank. Chips were respectively installed in the sampler.
6) 27 µL of DNB was loaded at L01-L04 using a wide mouth tip respectively, and allowed to stand at room temperature for more than half an hour.
7) PE150 sequencing reagent tank was prepared, HotMPS dNTP mixture and HotMPS dNTP mixture II in need were taken out 1 h in advance, and melted at room temperature for later use.
8) Adv Seqenzyme II was taken out and placed on ice for later use.
9) The samples were loaded as follows:

| Reagent | No. 1 pore | No. 2 pore | No. 15 pore |
|---|---|---|---|
| HotMP SdNTP mixture | 1500 µL | | / |
| HotMPS dNTP mixture II | | 1500 µL | / |
| Adv Seqenzyme II | 1000 µL | 1000 µL | / |
| MDA Reagent + MDA enzyme | / | / | 3875 µL +125 µL |

10) The reagents were mixed uniformly for later use.

### c. On-line sequencing

The reagent tank was placed in an instrument, a script was edited and PE150 sequencing was started.

### Experimental design:

| Example No. | Test conditions |
|---|---|
| 1 | scanning reagent with the addition of 10 mM IR13 |
| 2 | scanning reagent with the addition of 10 mM IR20 |
| 3 | scanning reagent with the addition of 10 mM IR22 |
| 4 | scanning reagent with the addition of 10 mM IR34 |
| 5 | scanning reagent with the addition of 5 mM IR41 |
| 6 | scanning reagent with the addition of 10 mM IR41 |
| 7 | scanning reagent with the addition of 20 mM IR41 |
| 8 | scanning reagent with the addition of 10 mM IR45 |

In all the above examples, the scanning reagent without the heteroaromatic ring compound was used as a blank control, wherein the scanning reagent is Tris buffer solution preloaded in the sequencing reagent tank - well No. 10 of the MGISEQ2000RS high-throughput (rapid) sequencing reagent kit.

PE150 sequencing was performed using the prepared DNB and the fluorochrome-labeled nucleotide (from FCL PE100 reagent kit) carrying a cleavable blocking group: first strand was sequenced first, followed by sequencing the second strand. The nucleotide carrying a cleavable blocking group had removable moieties located at the base-linked fluorophore and the ribose 3'-OH. In each cycle, after incorporation of the fluorochrome-labeled nucleotide into the complementary strand of the nucleic acid to be tested, a scanning reagent was pumped in for photographing; the imaging instrument used for photographing was MGISEQ-2000RS, the excitation wavelengths for irradiating fluorochrome were about 532 nm and 660 nm, and the exposure time was 30 milliseconds for 1-100 cycles, 40 milliseconds for 100-200 cycles, and 50 milliseconds for 200-300 cycles. After photographing, the fluorophore and the blocking group at the ribose 3'-OH were removed using THPP. The sequencing results are shown in Tables 1-6.

**Table 1**

| | Scanning reagent (blank control) | Scanning reagent with the addition of 10 mM IR13 |
|---|---|---|
| | | 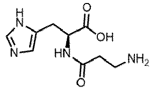 |
| First strand Q30 (%) | 97.01 | 97.17 |
| Second strand Q30 (%) | 91.48 | 93.52 |
| Q30 (%) | 94.25 | 95.35 |
| First strand error rate (%) | 0.14 | 0.12 |
| Second strand error rate (%) | 0.62 | 0.25 |
| Average error rate (%) | 0.38 | 0.18 |
| Signal rise value | 1.64 | 1.75 |

**Table 2**

| | Scanning reagent (blank control) | Scanning reagent with the addition of 10 mM IR20 |
|---|---|---|
| | | 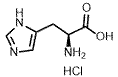 |
| First strand Q30 (%) | 97.01 | 97 |
| Second strand Q30 (%) | 91.48 | 91.7 |
| Q30 (%) | 94.25 | 94.35 |
| First strand error rate (%) | 0.14 | 0.12 |
| Second strand error rate (%) | 0.62 | 0.5 |
| Average error rate (%) | 0.38 | 0.31 |
| Signal rise value | 1.64 | 1.48 |

**Table 3**

| | Scanning reagent (blank control) | Scanning reagent with the addition of 10 mM IR22 |
|---|---|---|
| | | 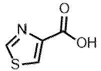 |
| First strand Q30 (%) | 96.39 | 97.07 |
| Second strand Q30 (%) | 91.21 | 91.36 |
| Q30 (%) | 93.8 | 94.35 |
| First strand error rate (%) | 0.14 | 0.12 |
| Second strand error rate (%) | 0.58 | 0.55 |
| Average error rate (%) | 0.36 | 0.34 |
| Signal rise value | 2.07 | 1.84 |

**Table 4**

| | Scanning reagent (blank control) | Scanning reagent with the addition of 10 mM IR34 |
|---|---|---|
| | | 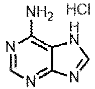 |
| First strand Q30 (%) | 97.13 | 97.11 |
| Second strand Q30 (%) | 91.97 | 92.64 |
| Q30 (%) | 94.55 | 94.88 |
| First strand error rate (%) | 0.11 | 0.11 |
| Second strand error rate (%) | 0.4 | 0.34 |
| Average error rate (%) | 0.26 | 0.22 |
| Signal rise value | 1.75 | 1.89 |

**Table 5**

| | Scanning reagent (blank control) | Scanning reagent with the addition of IR41 | | |
|---|---|---|---|---|
| | | 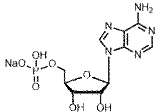 | | |
| | | 5 mM | 10 mM | 20 mM |
| First strand Q30 (%) | 97.13 | 96.9 | 97.5 | 96.71 |
| Second strand Q30 (%) | 91.97 | 93.31 | 94.27 | 92.82 |
| Q30 (%) | 94.55 | 95.1 | 95.88 | 94.77 |
| First strand error rate (%) | 0.11 | 0.12 | 0.1 | 0.13 |
| Second strand error rate (%) | 0.4 | 0.29 | 0.22 | 0.35 |
| Average error rate (%) | 0.26 | 0.2 | 0.16 | 0.24 |
| Signal rise value | 1.75 | 1.89 | 1.88 | 1.85 |

**Table 6**

| | Scanning reagent (blank control) | Scanning reagent with the addition of 10 mM IR45 |
|---|---|---|
| | | 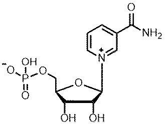 |
| First strand Q30 (%) | 97.13 | 95.16 |
| Second strand Q30 (%) | 91.97 | 92.03 |
| Q30 (%) | 94.55 | 93.6 |
| First strand error rate (%) | 0.11 | 0.23 |
| Second strand error rate (%) | 0.4 | 0.48 |
| Average error rate (%) | 0.26 | 0.35 |
| Signal rise value | 1.75 | 1.87 |

As shown in Tables 1-6, the tested heteroaromatic ring additives could all alleviate, to different degrees, deterioration of sequencing quality (a decrease in Q30, an increase in error rate, and a low signal rise value are considered to be deterioration of sequencing quality) due to DNA template damage. Among them, IR41, i.e., adenosine 5'-monophosphate monosodium salt, had the best effect in improving the sequencing quality.

As shown in Table 5, the PE150 sequencing was performed by adding adenosine 5'-monophosphate monosodium salt in the scanning reagent. Compared with the blank control group, the groups with scanning reagent with the addition of different concentrations of adenosine 5'-monophosphate monosodium salt exhibited a second strand Q30 (%) in the range of 92.82-94.27, being higher than 91.97 of the blank control; a second strand error rate (%) in the range of 0.22-0.35, being lower than 0.4 of the blank control; and a signal rise value in the range of 1.85-1.89, being higher than 1.75 of the blank control.

By comparing the sequencing quality of groups with different concentrations of adenosine 5'-monophosphate monosodium salt, it was found that 10 mM of adenosine 5'-monophosphate monosodium salt had the best improvement effect.

The above results show that the incorporation of different concentrations of the heteroaromatic ring compound (e.g., adenosine 5'-monophosphate monosodium salt) in scanning reagent can reduce the deterioration of sequencing quality due to the damage to DNA template and/or extension strand, and that the heteroaromatic ring compound can protect the nucleic acid strand such as DNA template or extension strand during nucleic acid detection.

The above examples are merely illustrative of the technical solutions of the invention, and not restrictive, and modifications and equivalent substitutions made thereto, without departing from the spirit and scope of the technical solutions of the invention, are intended to be included within the scope of the invention.

## Claims

1. Use of a heteroaromatic ring compound as a nucleic acid protective agent in nucleic acid detection, wherein the heteroaromatic ring compound is selected from a compound shown in formula (I) or an isomer, salt or N-oxide thereof, or a solvate (such as a hydrate) of a compound shown in formula (I) or an isomer, salt or N-oxide thereof; 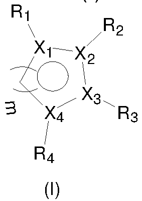
wherein, X₁, X₂, X₃ each are independently selected from C and N; X₄ is selected from N and S; and, at least one of X₁, X₂, X₃ and X₄ is N;
m is 1 or 2;
R₁ is absent or is selected from H, C₁₋₆ alkyl, amino, hydroxy, C₂₋₆ alkenyl, C₂₋₆ alkynyl, and C₁₋₆ alkoxy;
R₂ is absent or is selected from -(CH₂)ₙ-COOH or -(CH₂)ₙ-CONH₂; n is 0, 1, 2, 3 or 4; optionally, -(CH₂)ₙ- is substituted by amino; optionally, the amino is substituted by - (C=O)-(CH₂)_{q}-NH₂; q is 0, 1, 2, 3 or 4;
R₃ is absent or is H;
or, R₂ and R₃ form a 5- to 6-membered heteroaryl group comprising 1-2 nitrogen atoms, the heteroaryl is optionally substituted by 1 or 2 substituents, and the substituents each are independently selected from: hydroxy, amino, carbonyl, C₁₋₆ alkyl, C₂₋₆ alkenyl, C₂₋₆ alkynyl, and C₁₋₆ alkoxy;
R₄ is absent or is H, or is selected from the following structures:

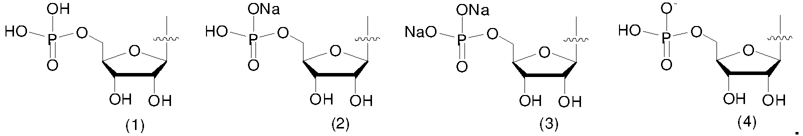

2. The use as claimed in claim 1, wherein the compound shown in the formula (I) has one of the following structures: 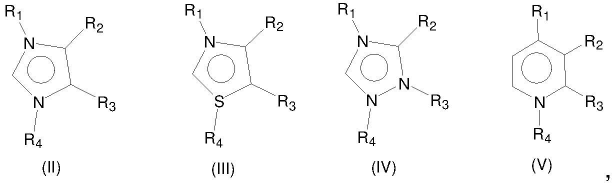
preferably, R₂ is selected from -(CH₂)ₙ-COOH or -(CH₂)ₙ-CONH₂; n is 0, 1 or 2;
optionally, -(CH₂)ₙ- is substituted by amino; optionally, the amino is substituted by -(C=O)-(CH₂)_{q}-NH₂; preferably, q is 0, 1 or 2;
preferably, R₄ is absent;
preferably, R₂ and R₃ form a 6-membered heteroaryl group (e.g. pyrimidinyl) comprising 1 or 2 nitrogen atoms; the heteroaryl is optionally substituted by 1 or 2 substituents, and the substituents each are independently selected from: hydroxy, amino, carbonyl, C₁₋₆ alkyl, C₂₋₆ alkenyl, C₂₋₆ alkynyl, and C₁₋₆ alkoxy.

3. The use as claimed in claim 1, wherein the compound shown in the formula (I) has one of the following structures: 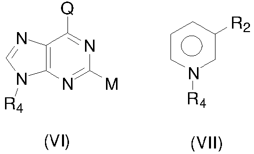
wherein, Q and M each are independently selected from: hydrogen, hydroxy, amino, carbonyl, C₁₋₆ alkyl, C₂₋₆ alkenyl, C₂₋₆ alkynyl, and C₁₋₆ alkoxy; preferably, Q and M each are independently selected from hydrogen, hydroxy, amino, and carbonyl;
preferably, R₄ is absent, or, R₄ has one of the structures shown in the formulas (1), (2), (3) and (4).

4. The use as claimed in any of claims 1-3, wherein the compound shown in the formula (I) is selected from the following compounds: 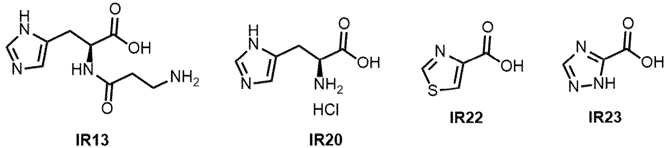 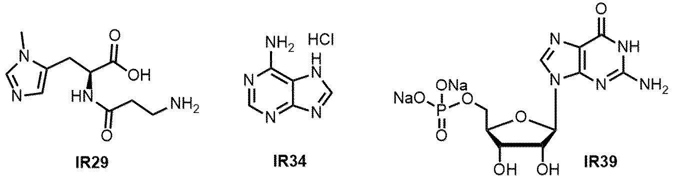 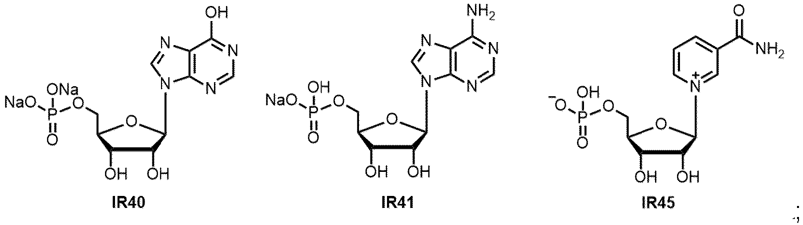 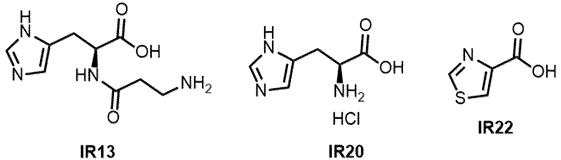 preferably, 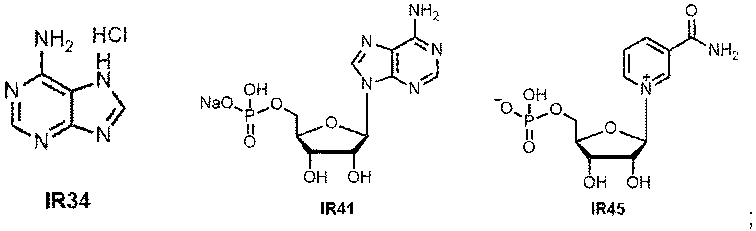 more preferably,

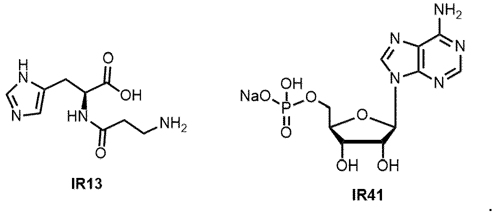

5. The use as claimed in any of claims 1-4, wherein the nucleic acid detection involves detecting a fluorescence signal; preferably, the fluorescence signal is generated by a light reaction or a bioluminescent reaction.

6. The use as claimed in claim 5, wherein the nucleic acid detection is sequencing, such as high-throughput sequencing, further such as SBS sequencing, ligation sequencing, hybridization sequencing, nanopore sequencing, or cPAL sequencing;
optionally, the nucleic acid detection is quantitative PCR.

7. A reagent, comprising one or more heteroaromatic ring compounds as defined in any of claims 1-4, and further comprising a Tris buffer solution;
preferably, the heteroaromatic ring compound in the reagent has a concentration of is 0.1-500 mM, more preferably 10 mM;
preferably, the reagent is a scanning reagent;
preferably, the heteroaromatic ring compound acts as a nucleic acid protective agent in nucleic acid sequencing.

8. A kit, comprising the reagent as claimed in claim 7;
preferably, the kit further comprises one or more additional reagents required for nucleic acid detection, e.g., a primer, a polymerase, a buffer solution, a wash solution, or any combination thereof;
preferably, the kit is used for sequencing;
more preferably, the kit further comprises a support for immobilizing the nucleic acid molecule to be sequenced, a reagent for immobilizing the nucleic acid molecule to be sequenced to the support and/or one or more cleavage reagents.

9. Use of the reagent as claimed in claim 7 or the kit as claimed in claim 8 for nucleic acid detection;
preferably, the nucleic acid detection is sequencing, such as high-throughput sequencing, further such as SBS sequencing, ligation sequencing, hybridization sequencing, nanopore sequencing, or cPAL sequencing;
preferably, the nucleic acid detection is quantitative PCR.

10. A method for inhibiting degradation of a nucleic acid in nucleic acid detection, which comprises, when detecting the nucleic acid to be tested in a reaction mixture, adding one or more heteroaromatic ring compounds as defined in any of claims 1-4 to inhibit degradation of the nucleic acid to be tested;
preferably, the test involves detecting a fluorescence signal, and the reaction mixture comprises a reactant that generates a fluorescence signal, a nucleic acid to be tested, and a buffer;
preferably, the fluorescence signal is generated by a light reaction or a bioluminescent reaction.

11. A method for detecting a target nucleic acid molecule, which comprises detecting the target nucleic acid molecule in a reaction mixture in the presence of one or more heteroaromatic ring compounds as defined in any of claims 1-4;
preferably, the detection involves detecting a fluorescence signal, and the reaction mixture comprises a reactant that generates a fluorescence signal, a target nucleic acid molecule, and a buffer;
preferably, the fluorescence signal is generated by a light reaction or a bioluminescent reaction.

12. A method for determining a target single-stranded polynucleotide sequence, which comprises:
(a) incorporating one or more nucleotides on a complementary strand of the target single-stranded polynucleotide sequence;
(b) providing a condition for an incorporated nucleotide to generate a fluorescence signal in the presence of one or more heteroaromatic ring compounds as defined in any of claims 1-4 and detecting the fluorescence signal to determine type of the incorporated nucleotide;
preferably, the method further comprises, (c) repeating steps (a) - (b) at least once;
preferably, the method further comprises, (d) determining the target single-stranded polynucleotide sequence;
preferably, the heteroaromatic ring compound has a concentration of greater than 0.1 mM, preferably 0.1-500 mM, more preferably 5-20 mM, and most preferably 10 mM.

13. The method as claimed in claim 12, wherein the incorporated nucleotide includes a sugar unit, a nucleobase, a phosphate group, and a cleavable blocking group to reversibly prevent further extension of the complementary strand of the target single-stranded polynucleotide sequence;
preferably, the nucleobase is selected from adenine (A), guanine (G), thymine (T), cytosine (C) and uracil (U);
preferably, each type of nucleotide is capable of generating a fluorescence signal that is distinguishable from each other to identify the type of the incorporated nucleotide;
preferably, the fluorescence signal is generated by a light reaction or a bioluminescent reaction;
preferably, the method further comprises, between (b) and (c): (b') removing from the incorporated nucleotide a moiety capable of generating a fluorescence signal directly or indirectly attached thereto; and/or, washing to remove an unincorporated nucleotide;
preferably, the method further comprises, between (b) and (c): (b") removing from the incorporated nucleotide a cleavable blocking group, to allow further extension of the complementary strand of the target single-stranded polynucleotide sequence;
preferably, the target single-stranded polynucleotide is present in a nucleic acid array; preferably, each site on the array comprises multiple copies of a single target single-stranded polynucleotide;
preferably, the nucleic acid array is immobilized on a solid support.

14. The method as claimed in claim 12 or 13, wherein providing a condition for the incorporated nucleotide to generate a fluorescence signal comprises: irradiating a reaction mixture comprising a fluorescently labeled nucleotide to generate a fluorescence signal; preferably, the fluorescently labeled nucleotide is obtained by linking a linker to a nucleotide;
preferably, the linker is acid labile, photolabile or comprises a disulfide bond.

15. The method as claimed in claim 14, wherein the method comprises: incorporating one or more fluorescently labeled nucleotides on the complementary strand of the target single-stranded polynucleotide sequence; irradiating a reaction mixture in the presence of one or more heteroaromatic ring compounds as defined in any of claims 1-4 to generate a fluorescence signal and detecting the fluorescence signal to determine the type of the incorporated nucleotide;
preferably, the method further comprises: removing from the incorporated nucleotide a fluorescent label attached thereto; and/or, washing to remove an unincorporated nucleotide;
preferably, the method further comprises: removing from the incorporated nucleotide the cleavable blocking group to allow further extension of the complementary strand of the target single-stranded polynucleotide sequence.

16. The method as claimed in claim 12 or 13, wherein providing a condition for the incorporated nucleotide to generate a fluorescence signal comprises: irradiating a reaction mixture to generate a fluorescence signal, wherein the reaction mixture comprises an unlabeled nucleotide and a fluorescently labeled affinity reagent that is capable of specifically binding to the unlabeled nucleotide;
preferably, the affinity reagent specifically binds to a base, sugar, cleavable blocking group, or combination of these components incorporated into the nucleotide;
preferably, the affinity reagent is antibody, aptamer, Affimer or Knottin; preferably, the affinity reagent is antibody.

17. The method as claimed in claim 16, wherein the method comprises: incorporating one or more unlabeled nucleotides on the complementary strand of the target single-stranded polynucleotide sequence; providing a fluorescently labeled affinity reagent and indirectly attaching a fluorescent label to the incorporated nucleotide via specific binding between the affinity reagent and the nucleotide; irradiating a reaction mixture in the presence of one or more heteroaromatic ring compounds as defined in any of claims 1-4 to generate a fluorescence signal and detecting the fluorescence signal to determine the type of the incorporated nucleotide;
preferably, the method further comprises: removing from the incorporated nucleotide the affinity reagent attached thereto; and/or, washing to remove an unincorporated nucleotide;
preferably, the method further comprises: removing from the incorporated nucleotide the cleavable blocking group to allow further extension of the complementary strand of the target single-stranded polynucleotide sequence.

18. The method as claimed in claim 12 or 13, wherein providing a condition for the incorporated nucleotide to generate a fluorescence signal comprises: allowing a reaction mixture to generate a fluorescence signal by a bioluminescent reaction, wherein the reaction mixture comprises a nucleotide carrying a tag, a luciferase capable of specifically binding to the tag and a substrate for the luciferase;
preferably, the tag carried by the nucleotide is a small molecule marker, e.g., selected from biotin, digoxin, N3G or FITC, and the luciferase carries a pairing member corresponding to the small molecule marker.

19. The method as claimed in claim 18, wherein the method comprises: incorporating one or more nucleotides carrying a tag on the complementary strand of the target single-stranded polynucleotide sequence; providing a luciferase attached with a pairing member capable of specifically binding to the tag and indirectly attaching the luciferase to the incorporated nucleotide via specific binding between the pairing members; providing a substrate for the luciferase in the presence of one or more heteroaromatic ring compounds as defined in any of claims 1-4 to generate a fluorescence signal and detecting the fluorescence signal to determine the type of the incorporated nucleotide;
preferably, the method further comprises: removing from the incorporated nucleotide the luciferase attached thereto; and/or, washing to remove an unincorporated nucleotide;
preferably, the method further comprises: removing from the incorporated nucleotide the cleavable blocking group to allow further extension of the complementary strand of the target single-stranded polynucleotide sequence.
